(19) **Europäisches Patentamt**

**European Patent Office**

**Office européen des brevets**

(11) **EP 1 102 760 B1**

(12) **EUROPEAN PATENT SPECIFICATION**

(45) Date of publication and mention
of the grant of the patent:
**17.03.2004 Bulletin 2004/12**

(21) Application number: **99940905.5**

(22) Date of filing: **05.08.1999**

(51) Int Cl.$^7$: **C07D 307/36**, B01J 23/887

(86) International application number:
**PCT/US1999/017758**

(87) International publication number:
**WO 2000/008008 (17.02.2000 Gazette 2000/07)**

(54) **PROCESS FOR THE OXIDATION OF BUTADIENE AND BUTENE WITH COBALT AND MOLYBDENUM OXIDE BASED CATALYSTS**

VERFAHREN ZUR OXIDATION VON BUTADIEN UND BUTEN MIT KATALYSATOREN AUF DER BASIS VON COBALT UND MOLYBDENUM OXID

PROCEDE D'OXYDATION DE BUTADIENE ET DE BUTENE AU MOYEN DE CATALYSEURS A BASE D'OXYDE DE COBALT ET DE MOLYBDENE

(84) Designated Contracting States:
**AT BE CH CY DE DK ES FI FR GB GR IE IT LI LU MC NL PT SE**

(30) Priority: **06.08.1998 US 95577 P**

(43) Date of publication of application:
**30.05.2001 Bulletin 2001/22**

(73) Proprietor: **E.I. DU PONT DE NEMOURS AND COMPANY**
**Wilmington Delaware 19898 (US)**

(72) Inventor: **KOURTAKIS, Kostantinos**
**Swedesboro, NJ 08085 (US)**

(74) Representative: **Carpmaels & Ransford**
**43 Bloomsbury Square**
**London WC1A 2RA (GB)**

(56) References cited:
**US-A- 3 894 055**        **US-A- 3 894 056**

• **DATABASE WPI Section Ch, Week 199723 Derwent Publications Ltd., London, GB; Class E17, AN 1997-253488 XP002124359 & JP 09 085095 A (SHOWA DENKO KK), 31 March 1997 (1997-03-31)**

EP 1 102 760 B1

**Description**

FIELD OF INVENTION

This invention concerns catalysts useful in the oxidation of hydrocarbons. The catalysts consist essentially of molybdenum, cobalt, oxygen, and an alkali, alkaline earth, or rare earth metal, and optionally containing nickel.

BACKGROUND

Furan is an important intermediate for the production of many commercial products. In particular, furan can be easily hydrogenated to tetrahydrofuran, used in many industrial polymers.
Molybdenum based catalysts have been widely used for the production of furan from butadiene. Most include vanadium phosphorus, bismuth or lead as co-catalysts or promoters (Japanese Patent Nos. 46-22007, 46-22009. and 47-43545, and U.S. Patent Nos. 3,894,055 4,309,355, and 3,894,056).
Alkali and lanthanide metals have been added to molybdenum based catalysts that are utilized in oxidation of butene or butadiene, however these have been added in order to increase the conversion or selectivity of the hydrocarbon to maleic anhydride (U.S. Patent Nos. 5,334,743,4,155,920,4,292,203, and 4,240,931, and Zielinski, et al., Polish J. of Chem., 57, 957 (1983)).
We have found that the addition of an alkali or lanthanide metal to molybdenum/cobalt based catalysts improves the selectivity and/or conversion to furan from the oxidation of butene or butadiene, with little or no maleic anhydride produced.

SUMMARY OF THE INVENTION

This invention comprises a process for the oxidation of alkenes and alkadienes having 4-10 carbon atoms to the corresponding furan compound by contacting the alkene or alkadiene with a source of oxygen in the presence of a catalyst consisting essentially of molybdenum, cobalt, oxygen, and at least one element selected from the group consisting of alkali metals and rare earth metals, and optionally containing nickel. Preferably the alkali metal is selected from the group consisting of K and Li, the rare earth metal is Eu and the alkenes and alkadienes are 1-butene and 1-3 butadiene.
Another preferred form of the catalyst is of the formula $Co_aNi_bMo_cQ_dO_y$, wherein Q is selected from the group consisting of alkali metals and rare earth metals; a is about 0.05 to about 2; b is 0 to about 2; c is about 0.5 to about 10; d is about 0.01 to about 5; and y is a number sufficient so that the oxygen present balances the charges of the other elements in the compound.

DETAILED DESCRIPTION OF THE INVENTION

The catalysts of the present invention are used in the production of furan compounds by the oxidation of 4-10 carbon alkenes and alkadienes. Preferred is the vapor phase oxidation of butene and butadiene to furan. The catalysts consist essentially of molybdenum, cobalt, oxygen, and at least one element selected from the group consisting of alkali metals and rare earth metals, and optionally containing nickel. These catalysts are more selective to furan from the oxidation of alkenes and alkadienes, and/or result in higher yields (conversions).
Alkali elements are hereby defined as any of the elements in Group 1A of the periodic table. Preferred is Li and K. Most preferred is Li.
Rare earth elements are hereby defined as any element of atomic number 57-71, also called lanthanides. Preferred is Eu.
A preferred form of the catalysts of the present invention is $Co_aNi_bMo_cQ_dO_y$, wherein Q is selected from the group consisting of alkali metals and rare earth metals; a is about 0.05 to about 2; b is 0 to about 2; c is about 0.5 to about 10; d is about 0.01 to about 5; and y is a number sufficient so that the oxygen present balances the charges of the other elements in the compound.
The catalysts of the present invention can be either a particular structure (containing a certain ratio of cations) or a combination of structures and thus comprise a mixture of the crystalline oxides of the catalyst compound of the formula given above, and may further comprise the amorphous phase of the compound.
The catalysts can be prepared by any method that results in a composition with the desired combination of elements, including coprecipitation, impregnation, sol-gel techniques, aqueous or nonaqueous solution or suspension mixing, freeze drying, spray roasting, spray drying or dry mixing. Small or trace amounts of elements other than the desired elements may be present in the final composition. Ceramic methods, i.e., solid state techniques could be used but are, in general, less preferred. Certain of the compounds are better prepared by one method over another as appreciated by one of ordinary skill in the art.

The catalysts are prepared at normal atmospheric pressure, but elevated or reduced pressures can be employed. Agitation is not required, but is usually provided to facilitate a homogeneous mix and to facilitate heat transfer.

One process for the preparation of a catalyst comprises contacting at least one cation containing compound with at least one other cation containing compound for each of the other cations of the final catalyst compound in a solution comprising water to form a resultant solution or colloid; freezing the resultant solution or colloid to form a frozen material; freeze drying the frozen material; and heating the dried frozen material to yield the catalytic compound.

A consequence of this method is that higher metal dispersion and uniformity can be achieved in the matrix than is normally attainable using more conventional synthetic methods. The use of metal oxides as matrices offers the opportunity to design and improve catalysts through surface structure, epitaxial effects, surface acidity, high metal dispersion and catalyst stability, and other key metal-matrix interactions. The catalysts may optionally be supported on conventional catalytic solid supports including but not limited to silica, zirconia, titania, zeolites, or mixtures thereof.

Methods to support the catalysts of the instant invention onto conventional catalytic supports include but are not limited to spray drying. Spray drying employs an aqueous slurry containing the catalyst and a solid support, also called a binder. The slurry is spray dried to form microspherical particles that are then calcined by heating. These particles, depending on choice of conditions and binder used, are particularly suitable for use in recirculating solids or riser reactors.

The solvent in the reaction mixtures can be removed in several different ways: conventional drying, freeze and vacuum drying, spray drying, or the solvent can be exchanged under supercritical conditions. Especially envisioned is the exchange of solvent using $CO_2$ gas under supercritical conditions, generating very high surface area materials (several hundred $m^2/g$ surface areas); however, solvent exchange to remove the water would be required before extraction. This would generate the catalysts of the present invention in an aerogel form.

The catalysts can optionally consist of a mixture of crystalline phases. For example, the $CoMo_3KO_x$ catalyst prepared in Example 1 consists of two major crystalline phases, beta cobalt molybdate ($CoMoO_4$) and potassium tetramolybdate ($K_2Mo_4O_{13}$), as evidenced by powder X-ray diffraction data, shown in Figure 1. Powder X-ray diffraction data were obtained using Phillips XRD 3600 powder diffractometer with CuK$\alpha$ radiation using 0.02 degree 2$\Theta$ steps, and 0.5 seconds residence time per step. Both phases, $\beta$- $CoMoO_4$ (21-0866) and $K_2Mo_4O_{13}$ (27-416), are identified by comparison to these phases listed from diffraction data in the International Centre for Diffraction Data, Swarthmore, PA. Each crystalline solid has its own characteristic X-ray powder pattern which may be used as a fingerprint for its identification. A combination of diffraction patterns of the two crystalline phases, $CoMoO_4$ and $K_2Mo_4O_{13}$ generates a pattern very similar to that shown in Figure 1, which was obtained from the catalyst composition $CoMo_4KO_x$. This indicates that these two crystalline phases are present in this material. Other amorphous phases may also be present.

Although the catalysts of the instant invention were used in a pulse reactor, it is to be understood that the invention is not limited to any particular type of reactor. The process described can be performed in any suitable reactor such as but limited to pulse. fluidized bed. fixed bed, steady state and riser reactors. A preferred reactor is a pulse reactor utilizing $O_2$ and temperatures of about 300-500°C.

A riser or transport line reactor is characterized by high gas velocities of from about 5 ft/s (about 1.5 m/s) to greater than 40 ft/s (12 m/s). Typically, the reactor line is vertically mounted with gas and solids flowing upward in essentially plug flow. The flow can also be downward and the reactor line can be mounted other than vertically. With upward flow of gas and solids, there can be a significant amount of local back mixing of solids, especially at the lower end of the velocity range. A fluidized bed reactor is characterized by extensive solids back mixing.

MATERIALS AND METHODS

Pulse reactor evaluation of the catalysts was carried out by injecting 0.05 ml pulses of hydrocarbon by means of a gas sampling valve contained in an oven at 170°C into a stream of helium flowing at 10 ml/min that passed over 0.5 grams of catalyst in a reactor made from 1/8 (3.2 mm) tubing, and heated in a tube furnace to 380°C. The effluent of the reactor passed through a thermal conductivity detector and then through a sample loop. When the pulse was in the sample loop, as determined by the thermal conductivity detector, it was injected into a gas chromatograph for analysis of the reaction products. After each hydrocarbon pulse, eight 0.5 ml pulses of 20% oxygen in helium were passed over the catalyst to reoxidize it. The gas chromatograph uses a 0.53 mm bore 25 m capillary Poroplot Q column and thermoconductivity detector. The reaction products were identified by a gas chromatograph mass spectrometer. An alternate method was to use a 0.5 ml pulse of a 10 parts of 20% oxygen in helium and 1 part hydrocarbon, indicated in the Feed column in Table 1. In this case 20% oxygen in helium was not fed between the hydrocarbon pulses.

Butane conversions and product selectivities are shown in Table 1. These were calculated by the following formulas

$$\% \text{ Butane conversion} = \text{butane in product/butane in feed} \times 100\%$$

$$\% \text{ Product selectivity} = \text{moles of product/moles butane reacted} \times 100\%$$

Stoichiometry was not analyzed for all Examples but was determined by calculation from amounts of reagents used.

EXAMPLE 1

$\underline{CoMo_3KO_x}$ (nominal stoichiometry)

8.82 g of ammonium molybdate, $(NH_4)_6Mo_7O_{24} \cdot 4H_2O$, (J.T. Baker, H30705) was added to 4.84 g of cobalt nitrate, $Co(NO_3)_2 \cdot 6H_2O$ (Aldrich, 03508KG) and 1.68 g of potassium nitrate, $K(NO_3)$ were added to 70 ml of $H_2O$ at room temperature. A red solution formed. The material was rapidly frozen at liquid nitrogen temperatures and evacuated by a freeze-drying process over several days. The material remained frozen during this evacuation procedure. The powder thus obtained was calcined in air to 400°C to produce the final catalyst

EXAMPLE 2

$\underline{Co_{0.5}Ni_{0.5}Mo_3LiO_x}$ (nominal stoichiometry)

8.87 g of ammonium molybdate, $(NH_4)_6Mo_7O_{24} \cdot 4H_2O$ (J.T. Baker, H30705), 1.96 g of nickel chloride hydrate, $NiCl_2 \cdot 6H_2O$, (Alfa,F10E13), 2.42 g of cobalt nitrate hexahydrate $(Co(NO_3)_2 \cdot 6H_2O$, J.T. Baker, 420559) and 2.29 g of lithium nitrate (Alfa, K091) were combined in 70 ml of water to obtain a reddish-brown solution. The material was rapidly frozen at liquid nitrogen temperatures an evacuated by a freeze-drying process over several days. The material remained frozen during this evacuation procedure. The powder thus obtained was calcined in air to 400°C to produce the final catalyst.

EXAMPLE 3

$\underline{Co_{0.5}Ni_{0.5}Mo_3EuO_x}$ (nominal stoichiometry)

8.86 g of ammonium molybdate, $(NH_4)_6Mo_7O_{24} \cdot 4H_2O$, (J.T. Baker, H30705), 1.96 g of nickel chloride hydrate $(NiCl_2 \cdot 6H_2O$, Alfa F10E13), 2.42 g of cobalt nitrate hydrate $(Co(NO_3)_2 \cdot 6H_2O$, J.T. Baker 420559), and 3.715 g of europium chloride (Aesar, R3853) were added to 70 ml of water. About 15 ml of HCl was added to partially dissolve some of the components. Upon addition of the hydrochloric acid, a red-pink colloid is transformed into a reddish-brown solution. The solution was rapidly frozen at liquid nitrogen temperatures and evacuated using a freeze-drying process over several days. The material remained frozen during this evacuation procedure. The free flowing powder thus obtained was calcined in air to 400°C for 5 hrs.

COMPARATIVE EXAMPLE A

$\underline{Co_{0.5}Ni_{0.5}Mo_3O_x}$

91.068 g of molybdenum pentachloride (Alfa, B13F38), 7.213 g of cobalt dichloride (Alfa, L15DO3), 7.207 g of nickel dichloride (Alfa, H22E24) were combined in 800 g (approximately 978 ml) of neopentyl alcohol (Aldrich, N720-6). The materials were loaded into a 2 liter round-bottom flask and refluxed for approximately 24 hours. After this 24 hour treatment, a bluish-green colloid formed. The material was rapidly frozen at liquid nitrogen temperatures and evacuated in a freeze-drying process over several days to produce a free-flowing powder. The material remained frozen during this evacuation procedure. The powder thus obtained was calcined in air to 350°C for four hours.

| TABLE I | | | | | | | | |
|---|---|---|---|---|---|---|---|---|
| | | | | | % Selectivity to | | | |
| Ex. | Composition | Feed | Temp. °C | Conversion % | Furan | BTX | Acrolein | MA |
| 1 | $CoMo_3O_xK$ | Bd | 440 | 17 | 12.5 | 7.6 | 4.3 | - |
| 2 | $Co_{0.5}Ni_{0.5}Mo_3O_xLi$ | Bd | 460 | 32 | 9.4 | 11 | 2.4 | - |
| | | Bd/$O_2$ | 400 | 24 | 11.7 | 0.8 | 2.9 | - |
| 3 | $Co_{0.5}Ni_{0.5}Mo_3O_xEu$ | Bd | 428 | 67 | 3.3 | 4.5 | - | 3 |
| | | 1-Bt | 440 | 25 | 3.9 | 0.7 | - | 6 |
| A | $Co_{0.5}Ni_{0.5}Mo_3O_x$ | Bd | 470 | 43 | 6.4 | 15 | - | - |
| | | 1-Bt | 473 | 33 | 4.8 | 14 | - | - |

## List of abbreviations

Bd    butadiene

1-Bt    1-butene

BTX    benzene-toluene-xylenes-styrene mixture

MA    maleic anhydride

**Claims**

1. A process for the oxidation of alkenes and alkadienes having 4-10 carbon atoms to the corresponding furan compound, comprising:

   contacting the alkene or alkadiene with a source of oxygen in the presence of a catalyst consisting essentially of molybdenum, cobalt, oxygen, and at least one element selected from the group consisting of alkali metals and rare earth metals, and optionally containing nickel.

2. The process of Claim 1 wherein the catalyst is of the formula $Co_aNi_bMo_cQ_dO_y$, wherein
   Q is selected from the group consisting of alkali metals and rare earth metals;
   a is about 0.05 to about 2;
   b is 0 to about 2;
   c is about 0.5 to about 10;
   d is about 0.01 to about 5; and
   y is a number sufficient so that the oxygen present balances the charges of the other elements in the compound.

3. The process of Claim 1 wherein the alkali metal is selected from the group consisting of K and Li.

4. The process of Claim 1 wherein the rare earth metal is Eu.

5. The process of Claim 1 wherein the alkenes and alkadienes are 1-butene and 1-3 butadiene.

6. The process of Claim 2 wherein Q is selected from the group consisting of K, Li, and Eu.

7. The process of Claim 1 wherein the catalyst is supported on a catalyst support.

**Patentansprüche**

1. Verfahren für die Oxidation von Alkenen und Alkadienen mit 3-10 Kohlenstoffatomen zu der entsprechenden Furanverbindung, umfassend:

   Inkontaktbringen des Alkens oder Alkadiens mit einem Ausgangsstoff von Sauerstoff in Anwesenheit eines Katalysators, bestehend im wesentlichen aus Molybdän, Cobalt, Sauerstoff und mindestens einem Element, ausgewählt aus der Gruppe, bestehend aus Alkalimetallen und Seltenerdmetallen, und gegebenenfalls enthaltend Nikkel.

2. Verfahren nach Anspruch 1, wobei der Katalysator die Formel $Co_aNi_bMo_cQ_dO_y$ hat, wobei
   Q aus der Gruppe, bestehend aus Alkalimetallen und Seltenerdmetallen, ausgewählt ist;
   a etwa 0,05 bis etwa 2 ist;
   b 0 bis etwa 2 ist;
   c etwa 0,5 bis etwa 10 ist;
   d etwa 0,01 bis etwa 5 ist; und
   y eine Zahl ist, ausreichend, so daß der vorhandene Sauerstoff die Ladungen der anderen Elemente in der Verbindung ausgleicht.

3. Verfahren nach Anspruch 1, wobei das Alkalimetall aus der Gruppe, bestehend aus K und Li, ausgewählt ist.

4. Verfahren nach Anspruch 1, wobei das Seltenerdmetall Eu ist.

5. Verfahren nach Anspruch 1, wobei die Alkene und Alkadiene 1-Buten und 1,3-Butadien sind.

6. Verfahren nach Anspruch 2, wobei Q aus der Gruppe, bestehend aus K, Li und Eu, ausgewählt ist.

7. Verfahren nach Anspruch 1, wobei der Katalysator auf einem Katalysatorträger geträgert ist.

**Revendications**

1. Procédé pour l'oxydation d'alcènes et d'alcadiènes contenant 3-10 atomes de carbone en composé de furanne correspondant, comprenant:

   la mise en contact de l'alcène ou de l'alcadiène avec une source d'oxygène en présence d'un catalyseur constitué essentiellement de molybdène, de cobalt, d'oxygène et d'au moins un élément choisi dans le groupe constitué de métaux alcalins et de métaux des terres rares, et éventuellement contenant du nickel.

2. Procédé suivant la revendication 1, dans lequel le catalyseur est de la formule $Co_aNi_bMo_cQ_dO_y$, dans laquelle:

   Q est choisi dans le groupe constitué de métaux alcalins et de métaux des terres rares;
   a est d'environ 0,05 à environ 2;
   b est de 0 à environ 2;
   c est d'environ 0,5 à environ 10;
   d est d'environ 0,01 à environ 5; et
   y est un nombre suffisant pour que l'oxygène présent équilibre les charges des autres éléments dans le composé.

3. Procédé suivant la revendication 1, dans lequel le métal alcalin est choisi dans le groupe constitué de K et de Li.

4. Procédé suivant la revendication 1, dans lequel le métal des terres rares est Eu.

5. Procédé suivant la revendication 1, dans lequel les alcènes et les alcadiènes sont le 1-butène et le 1,3-butadiène.

6. Procédé suivant la revendication 2, dans lequel Q est choisi dans le groupe constitué de K, de Li et de Eu.

7. Procédé suivant la revendication 1, dans lequel le catalyseur est supporté sur un support de catalyseur.